# EUROPEAN PATENT SPECIFICATION

(11) **EP 1 281 414 B1**
(45) Date of publication and mention of the grant of the patent: **01.09.2004**
(21) Application number: 02254477.9
(22) Date of filing: 26.06.2002
(51) Int. Cl.: A61M 16/04

(54) **Introducers and assemblies**
Einführungsvorrichtungen sowie Anordnungen
Dispositif d' introduction et ses éléments

(30) Priority: 04.08.2001 GB 0119124
(43) Date of publication of application: 05.02.2003
(73) Proprietor: Smiths Group PLC, London, NW11 8DS (GB)
(72) Inventor: Turnbull, Christopher Stratton, Hythe, Kent CT21 5RA (GB)
(74) Representative: Flint, Jonathan McNeill

(56) References cited:
- EP-A- 0 371 752
- GB-A- 2 310 605
- US-A- 4 637 388
- US-A- 4 960 122
- US-A- 5 222 487

## Description

This invention relates to introducers of the kind for a medical tube.

Tracheostomy tubes may be inserted into position in the trachea using an introducer inserted within the tube. The introducer has a pointed end protruding from the patient end of the tube to ease insertion of the tip of the tube through the tissues of the surgical opening into the trachea. The introducer also provides additional stiffness to prevent bending of the tube as a result of the forces used to push it into position. The introducer may protrude from the machine end of the tube sufficiently to form a handle by which the assembly can be gripped. Often, the tracheostomy tube is inserted along a guidewire or a small-bore catheter previously positioned using a guidewire. Where this technique is used, the introducer has a passage along its length along which the guidewire and catheter can extend. Examples of introducers are described in EP0590084, GB2224213, GB2341102 and GB2350061. US 4960122 describes an obturator for use in replacing a tracheal tube. The obturator is solid and has a flexible tip.

It is an object of the present invention to provide an alternative introducer and assembly including an introducer.

According to the present invention there is provided an introducer of the above-specified kind, characterised in that the introducer is tubular having a passage extending along its entire length, that the introducer has a tubular first end arranged to protrude from a first, patient end of the tube by a distance at least twice the external diameter of the tube with the passage opening at the tip of the first end, a relatively rigid midsection arranged to extend within the tube and a second end arranged to protrude from a second end of the tube, and that the first end is flexible and provides a flexible leader for introduction of the tube along a guidewire extending along the passage.

The second end of the introducer may be rigid. The introducer is preferably of plastics material and has a metal insert extending along the midsection. The second end and the remainder of the introducer are preferably of different materials. The second end and the midsection may be joined with one another by moulding. The midsection is preferably curved along its length. The first end is preferably tapered. The introducer may have a projection on its surface at a location along the midsection.

An introducer for a tracheostomy tube and an assembly of an introducer and tracheostomy tube will now be described, by way of example, with reference to the accompanying drawing, in which:
Figure 1 is a sectional side elevation view of the assembly; and
Figure 2 is an enlarged transverse sectional view of the assembly along the line II-II.

The assembly comprises a tracheostomy tube 1 and an introducer 2 inserted within the tube.

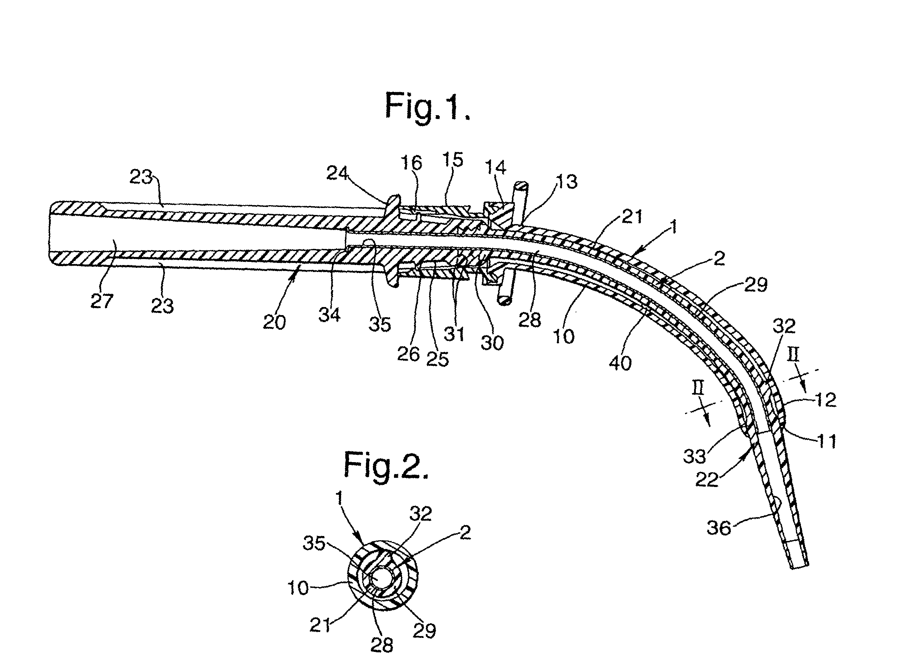

The tracheostomy tube 1 is of conventional form having a tubular shaft 10 moulded from a bendable plastics material and having an atraumatic, slightly tapered tip 11 at its patient end 12. The shaft 10 is curved along its length although its natural curve has a smaller radius than shown in Figure 1 where the tube has been straightened slightly by the introducer 2. At its rear or machine end 13, the tube 1 has a flange 14 moulded integrally with the shaft 10 and a female 15mm ISO tapered coupling 15 of a harder plastics material joined with the flange.

The introducer 2 comprises three sections along its length: a rear or machine-end handle 20, a midsection 21 and a forward or patient-end section 22. The handle 20 is straight and substantially cylindrical, being about 90mm long and 13 mm in diameter. Four gripping ribs 23 extend longitudinally along the handle and terminate towards its forward end at an annular flange 24 projecting radially outwardly. The diameter of the flange 24 is greater than the external diameter of the coupling 15 so that it abuts the end of the coupling when the introducer 2 is inserted in the tube 1. Forwardly of the flange 24, the handle 20 has a short, slightly tapered region 25 with a second, smaller flange 26. The dimensions of the tapered region 25 are such that it can extend freely within the coupling 15. The diameter of the second flange 26 is such that it catches on the forward side of a shallow internal lip 16 within the coupling. A passage or bore 27 extends along the length of the handle 20, the bore being tapered from its rear end until just rearwardly of the larger flange 24. The handle 20 is moulded from a hard plastics material, such as ABS, so that it is rigid

The forward end of the handle 20 is moulded about the rear end of a stainless steel tube insert 28, which extends forwardly along the midsection 21. An outer shaft 29 of a soft plastics material such as polyurethane extends along the outside of the insert 28 beyond its forward end to form the patient-end section 22. At its rear end 30, the plastics shaft 29 is shaped with formations that key it into the forward end of the handle 20. The midsection 21 is curved along its length and is rigid because of the presence of the metal insert 28. The midsection 21 has a circular section with an external diameter slightly less than the internal diameter of the tube 1. The midsection 21 may have an optional surface formation 32 close to its forward or patient end. The surface formation takes the form of a hemispherical bump or projection 32 on the external surface on the outside of the curve. The purpose of this projection 32 will be described later. The midsection 21 may also or alternatively have a shallow external annular rib 40 about halfway along its length, to reduce friction with the inside of the tube 1.

The patient-end section or tip 22 is a continuation of the plastics shaft 29. The rear end 33 of the tip 22 is enlarged slightly to be equal to or greater than the internal diameter of the tube 1 and it tapers along its length to a reduced diameter of about 3mm. The tip 22 is about 33mm long, it is straight and all but the rear 3mm or so of the tip projects from the tube 1. The length of flexible tip 22 projecting from the tube 1 is equal to about three to four times the external diameter of the shaft 29 and is at least twice the external diameter of the tube, preferably being about three times its diameter. The total length of the flexible tip 22 is, therefore, greater than about three times the diameter at its largest part.

The bore 27 along the handle 2 opens into the rear of the metal insert 28 via a small external flare 34 on the insert and forms a continuation with the bore 35 along the insert. The bore 35 of the insert 28 opens at its forward end into a bore 36 along the tip 22.

The shaft 29 is moulded first and then assembled onto the metal insert 28. The handle 20 is then moulded about the rearwardly projecting end of the insert 28 and the rear end 30 of the shaft 29. This ensures a good bond between the different components of the introducer.

The radius of curvature of the midsection 21 of the introducer 2 is selected to be the best for insertion of the tracheostomy tube 1 and is larger than the natural curvature of the tube, that is, without the introducer. The natural curvature of the tracheostomy tube 1 is chosen to be the best for the anatomy of the patient after insertion. The introducer 2, therefore, slightly straightens the tube 1 when it is inserted. When fully inserted, the rear flange 24 abuts the rear end of the coupling 15 and the forward flange 26 catches on the forward side of the lip 16, thereby resisting slightly removal of the introducer 2. The rear 33 of the patient end 22 locates as a close fit within the patient end 12 of the tube 1 so that there is a substantially smooth, stepless transition between the introducer 2 and the tube. Depending on the shape of the introducer 2 and the tube 1, the action of straightening a tube during insertion of an introducer may tend to distort the tube from a circular to a slightly oval section. This is undesirable because it forms a gap between the tip of the tube and the introducer, which could catch on tracheal tissue during insertion. The projection 32, where present, serves to reduce the force between the introducer 2 and the tip of the tracheostomy tube 1 by deflecting the tube at a point sufficiently behind the tip to avoid deformation of the circular opening at the end of the tube. The need for the projection 32 can be avoided by altering the curvature of the introducer at the forward end of the midsection.

The assembly of the introducer 2 and tube 1 is used in the usual way in conjunction with a guidewire and a small-bore catheter (not shown) having one end located in the patient's trachea. The assembly of the tracheostomy tube on the introducer is threaded over the catheter and the assembly is slid forwardly so that the patient end of the assembly passes smoothly through the tracheostomy into the trachea. The long, flexible tip 22 of the introducer 2 forms a leader that ensures the assembly follows the guidewire and catheter to the correct location within the trachea.

It will be appreciated that the invention is not confined to tracheostomy tubes but could be used with other medical tubes.

## Claims

1. An introducer (2) for a medical tube (1), **characterised in that** the introducer (2) is tubular having a passage extending along its entire length, that the introducer has a tubular first end (22) arranged to protrude from a first, patient end (12) of the tube by a distance at least twice the external diameter of the tube (1) with the passage opening at the tip of the first end (22), a relatively rigid midsection (21) arranged to extend within the tube (1) and a second end (20) arranged to protrude from a second end (13) of the tube, and that the first end (22) is flexible and provides a flexible leader for introduction of the tube along a guidewire extending along the passage.

2. An introducer according to Claim 1, **characterised in that** the second end (20) of the introducer (2) is rigid.

3. An introducer according to Claim 1 or 2, **characterised in that** the introducer (2) is of plastics material and has a metal insert (28) extending along the midsection (21).

4. An introducer according to any one of the preceding claims, **characterised in that** the second end (20) and the remainder (21, 22) of the introducer (2) are of different materials.

5. An introducer according to any one of the preceding claims, **characterised in that** the second end (20) and the midsection (21) are joined with one another by moulding.

6. An introducer according to any one of the preceding claims, **characterised in that** the midsection (21) is curved along its length.

7. An introducer according to any one of the preceding claims, **characterised in that** the first end (22) is tapered.

8. An introducer according to any one of the preceding claims, **characterised in that** the introducer (2) has a projection (32, 40) on its surface at a location along the midsection (21).

## Patentansprüche

1. Einführungsvorrichtung (2) für einen medizinischen Tubus (1), **dadurch gekennzeichnet, dass** die Einführungsvorrichtung (2) rohrförmig ist und eine sich über ihre gesamte Länge erstreckenden Kanal hat, dass die Einführungsvorrichtung ein rohrförmiges erstes Ende (22), das angeordnet ist, um von einem ersten, patientenseitigen Ende (12) des Tubus um eine Distanz vom mindestens zweifachen des äußeren Durchmessers des Tubus (1) herauszustehen, wobei der Kanal sich an der Spitze des ersten Endes (22) öffnet, einen relativ steifen Mittelabschnitt (21), der angeordnet ist, um sich innerhalb des Tubus (1) zu erstrecken, und eine zweites Ende (20), das angeordnet ist, um von einem zweiten Ende (13) des Tubus herauszustehen, hat, und dass das erste Ende (22) flexibel ist und eine flexible Führung zum Einführen des Tubus entlang eines sich entlang des Kanals erstreckenden Führungsdrahts bereitstellt.

2. Einführungsvorrichtung nach Anspruch 1, **dadurch gekennzeichnet, dass** das zweite Ende (20) der Einführungsvorrichtung (2) steif ist.

3. Einführungsvorrichtung nach Anspruch 1 oder 2, **dadurch gekennzeichnet, dass** die Einführungsvorrichtung (2) aus Kunststoffmaterial ist und einen metallenen Einsatz (28) hat, der sich entlang des Mittelabschnitts (21) erstreckt.

4. Einführungsvorrichtung nach einem der vorangehenden Ansprüche, **dadurch gekennzeichnet, dass** das zweite Ende (20) und der Rest (21, 22) der Einführungsvorrichtung (2) aus unterschiedlichen Materialien sind.

5. Einführungsvorrichtung nach einem der vorangehenden Ansprüche, **dadurch gekennzeichnet, dass** das zweite Ende (20) und der Mittelabschnitt (21) miteinander durch Verformen [moulding] verbunden sind.

6. Einführungsvorrichtung nach einem der vorangehenden Ansprüche, **dadurch gekennzeichnet, dass** der Mittelabschnitt entlang seiner Länge gebogen ist.

7. Einführungsvorrichtung nach einem der vorangehenden Ansprüche, **dadurch gekennzeichnet, dass** das erste Ende (22) konisch zulaufend ist.

8. Einführungsvorrichtung nach einem der vorangehenden Ansprüche, **dadurch gekennzeichnet, dass** die Einführungsvorrichtung (2) einen Vorsprung (32, 40) auf ihrer Oberfläche an einer Stelle entlang des Mittelabschnitts (21) hat.

## Revendications

1. Introducteur (2) pour un tube médical (1), **caractérisé en ce que** l'introducteur (2) est tubulaire et comporte un passage s'étendant le long de sa longueur totale, **en ce que** l'introducteur comporte une première extrémité tubulaire (22) agencée pour dépasser d'une première extrémité patient (12) du tube d'une distance au moins deux fois égale au diamètre externe du tube (1), le passage débouchant au bout de la première extrémité (22), une section médiane (21) relativement rigide agencée pour s'étendre à l'intérieur du tube (1) et une seconde extrémité (20) agencée pour dépasser d'une seconde extrémité (13) du tube, et **en ce que** la première extrémité (22) est flexible et fournit un guide flexible pour l'introduction du tube le long d'un guide souple s'étendant le long du passage.

2. Introducteur selon la revendication 1, **caractérisé en ce que** la seconde extrémité (20) de l'introducteur (2) est rigide.

3. Introducteur selon la revendication 1 ou 2, **caractérisé en ce que** l'introducteur (2) est réalisé en matière plastique et comporte un insert métallique (28) s'étendant le long de la section médiane (21).

4. Introducteur selon l'une quelconque des revendications précédentes, **caractérisé en ce que** la seconde extrémité (20) et le reste (21, 22) de l'introducteur (2) sont réalisés en matériaux différents.

5. Introducteur selon l'une quelconque des revendications précédentes, **caractérisé en ce que** la seconde extrémité (20) et la section médiane (21) sont jointes l'une à l'autre par moulage.

6. Introducteur selon l'une quelconque des revendications précédentes, **caractérisé en ce que** la section médiane (21) est courbée le long de sa longueur.

7. Introducteur selon l'une quelconque des revendications précédentes, **caractérisé en ce que** la première extrémité (22) est conique.

8. Introducteur selon l'une quelconque des revendications précédentes, **caractérisé en ce que** l'introducteur (2) comporte une partie saillante (32, 40) sur sa surface à un endroit le long de la section médiane (21).
